# EUROPEAN PATENT SPECIFICATION

(11) **EP 2 265 267 B1**
(45) Date of publication and mention of the grant of the patent: **21.09.2016**
(21) Application number: 09729990.3
(22) Date of filing: 10.04.2009
(51) Int. Cl.: A61K 31/14, A61K 31/185, A61K 31/198, A61K 31/205, A61K 31/452, A61K 31/662, A61K 31/69, A61K 33/02, A61P 11/00, A61P 31/00

(54) **COMPOSITIONS COMPRISING N-HALOGENATED OR N, N-DIHALOGENATED AMINE FOR TREATMENT AND PROPHYLAXIS OF BRONCHOPULMONARY INFECTIONS**
ZUSAMMENSETZUNGEN MIT N-HALOGENIERTEM ODER N, N-DIHALOGENIERTEM AMIN ZUR BEHANDLUNG UND PROPHYLAXE VON BRONCHOPULMONALEN INFEKTIONEN
COMPOSITIONS COMPRENANT UNE AMINE N-HALOGÉNÉE OU N,N-DIHALOGÉNÉE POUR LE TRAITEMENT ET LA PROPHYLAXIE DES INFECTIONS BRONCHO-PULMONAIRES

(30) Priority: 10.04.2008 US 43791 P
(43) Date of publication of application: 29.12.2010
(73) Proprietor: Gottardi, Waldemar, 6020 Innsbruck (AT); Nagl, Markus, 6094 Axams (AT)
(72) Inventor: GOTTARDI, Waldemar, 6020 Innsbruck (AT); NAGL, Markus, 6094 Axams (AT); NAJAFI, Ramin, Emeryville, CA 94608 (US); WANG, Lu, Emeryville CA 94608 (US); JAIN, Rakesh, K., Fremont CA 94539 (US); SHIAU, Timothy, P., Oakland CA 94602 (US); LOW, Eddy, Foster City CA 94404 (US); FRANCAVILLA, Charles, Fremont CA 94555 (US)
(74) Representative: Grund, Martin
(86) International application number: PCT/US2009/040251
(87) International publication number: WO 2009/126912

(56) References cited:
- WO-A-02/22118
- WO-A-2005/020896
- WO-A-2006/081392
- WO-A-2007/044559
- WO-A-2008/083347
- WO-A-2008/134687
- WO-A-2008/134692
- US-A- 3 950 536
- PINNA A ET AL: "Tolerability of N-chlorotaurine in the bronchopulmonary system" IJMM INTERNATIONAL JOURNAL OF MEDICAL MICROBIOLOGY, vol. 298, no. Suppl. 45, September 2008 (2008-09), page 56, XP008107727 & 60TH ANNUAL MEETING OF THE DEUTSCHEN-GESELLSCHAFT-FUR-HYGIENE-UND-MIK ROBIOLOGIE; DRESDEN, GERMANY; SEPTEMBER 21 -24, 2008 ISSN: 1438-4221
- GOTTARDI ET AL: "N-Chlorotaurine and ammonium chloride: An antiseptic preparation with strong bactericidal activity" INTERNATIONAL JOURNAL OF PHARMACEUTICS, ELSEVIER BV, NL, vol. 335, no. 1-2, 28 March 2007 (2007-03-28), pages 32-40, XP022003885 ISSN: 0378-5173
- WITKO-SARSAT VERONIQUE ET AL: "Neutrophil-derived long-lived oxidants in cystic fibrosis sputum" AMERICAN JOURNAL OF RESPIRATORY AND CRITICAL CARE MEDICINE, vol. 152, no. 6 PART 1, 1995, pages 1910-1916, XP008108007 ISSN: 1073-449X
- SANTANGELO F ET AL: "Taurine and the lung : which role in asthma?" ADVANCES IN EXPERIMENTAL MEDICINE AND BIOLOGY, SPRING STEET, NY, USA, vol. 526, no. Taurine 5, 1 January 2003 (2003-01-01), pages 403-410, XP008108011 ISSN: 0065-2598
- GSTOETTNER M ET AL: "REFRACTORY RHINOSINUSITIS COMPLICATING IMMUNOSUPPRESSION: APPLICATION OF N-CHLOROTAURINE, A NOVEL ENDOGENOUS ANTISEPTIC AGENT" ORL, BASEL, vol. 65, no. 5, 1 September 2003 (2003-09-01), pages 303-305, XP009065206 ISSN: 0301-1569

## Description

This application claims priority under 35 U.S.C. § 119 to U.S. Provisional Patent Application 61/043,791 filed April 10, 2008.

### FIELD

The invention relates to antimicrobial, anti-infective, and disinfecting compositions for the treatment and prophylaxis of bronchopulmonary infections. The invention relates particularly to certain N-halogenated or N,N-dihalogenated amine compositions for the treatment and prophylaxis of bronchopulmonary infections in mammals, particularly humans.

### BACKGROUND

Infections are a permanent problem in human medicine. Airway infections including bronchitis and pneumonia are mostly caused by viruses and bacteria, but in the last decades also fungal infections have been increasing mainly due to immunosuppressive drugs which are used to treat cancer.

Many antibiotics are available to treat bacterial infections, and fewer drugs to treat viral and fungal infections. A problem with antibiotics, particularly with antiviral and antifungal drugs, is antimicrobial resistance. Some bacteria contain multidrug resistance genes and can be treated successfully only with a few available antibiotics. Recently, such genes have been found in fungi too, and a significant number of immunosuppressed patients die from fungal infections which are acquired through the airway route. To date, only a few viruses can be treated with specific antiviral agents.

Antiseptics are active against a broad-spectrum of pathogens usually including all mentioned classes of pathogens. If antiseptics are used in sufficient concentrations, acquired resistance (i.e., resistance which develops during repeated application of the same substance) does not occur. This is due to the unspecific reaction mechanism of most antiseptics, and is an advantage above antibiotics. However, antiseptics are much more toxic than antibiotics so that they cannot be applied systemically (i.e., intravenously, orally, intra-arterially). Only topical application to skin and mucous membranes is possible. There are delicate locations where not all antiseptics can be applied, e.g., the eye, body cavities such as paranasal sinuses, and the urinary bladder.

Special areas are the deeper airways, particularly the bronchopulmonary system, consisting of bronchi, bronchioli, and alveoli. Antiseptics are not used upon inhalation since significant concentrations are not tolerated because of irritative or toxic reactions, or considerable systemic resorption (e.g., alcohols, iodine). For instance, chloramine T has been shown to cause asthmatic and inflammatory symptoms after inhalative application.

WO 2006/081392 A1 discloses the use of N-halogenated and N,N-dihalogenated amino acids and ammonium salts thereof in the treatment of pulmonary infections. WO 2005/020896 A2 discloses the use of N,N-dihalogenated amino acids and ammonium salts thereof in the treatment of pulmonary infections. WO 2007/044559 A1 discloses the use of N-halogenated and N,N-dihalogenated amino acids and ammonium salts thereof in the treatment of ventilator-associated pneumonia. WO 02/22118 A1 discloses a composition containing N-chlorotaurine for treating bronchitis and pneumonia caused by fungal infections. Witko-Sarsat et al. (1995), Am J Respir Crit Care Med ; 152(6 Pt 1):1910-6, describes that increased levels of chloramines are associated with better respiratory scores in patients with cystic fibrosis.

Therefore, new compositions are needed for the treatment of infections of the bronchopulmonary system which can be applied topically, have sufficient activity against the causative pathogens, do not induce resistance of the pathogens, and are well tolerated upon inhalation.

### SUMMARY

The invention is defined by the appended claims.

In one aspect disclosed is a composition for the treatment or prophylaxis of a bronchopulmonary infection in a mammal, the composition comprising a therapeutically effective amount of one or more N-halogenated or N,N-dihalogenated amines or mixtures thereof. As used herein in all aspects, the terms "N-halogenated amine(s)" and "N,N-dihalogenated amine(s)" include analogues and derivatives thereof, and pharmaceutically acceptable salts or esters of any of the foregoing compounds. As used herein, in certain embodiments, the clause "N-halogenated amine and N,N-dihalogenated amine" also include a mixture thereof.

In various embodiments, the halogen of the N-halogenated or N,N-dihalogenated amine is chlorine. The halogen could be any group 17 element such as fluorine, bromine or iodine.

An N-halogenated or N,N-dihalogenated amine can be a derivative of a protein, peptide, or amino acid, or pharmaceutically acceptable salts thereof. Additionally, the amine can be derived from at least one of an α-amino carbonic acid (also referred to herein as α-amino acid or α-amino carboxylic acid, such as glycine, alanine, leucine), a β-amino carbonic acid (e.g., β-alanine), an α-amino sulfonic acid (e.g., aminomethane sulfonic acid), a β -amino sulfonic acid (e.g., taurine and its derivatives alkylated at a carbon, e.g., dimethyltaurine), and an aliphatic amine (e.g., ethylamine). In certain embodiments, the N-halogenated or N,N-dihalogenated amine can be N-chlorotaurine (NCT) or N,N-dichlorotaurine (NDCT), or a mixture thereof, or pharmaceutically acceptable salts or esters thereof, e.g. an alkali salt, such as the sodium salt.

Compositions of the disclosure comprise one or more N-halogenated or N,N-dihalogenated amines at a concentration of about 0.001% to about 10% weight per volume, e.g. about 0.1% to about 5%, e.g. about 0.5% to about 1% active ingredient.

In certain embodiments, the compositions comprise one or more N-halogenated or N,N-dihalogenated amines in combination with an ammonium salt, e.g. ammonium chloride. One example is a combination of NCT and/or NDCT (or their derivatives), with ammonium chloride. Each of the constituents of the combined composition (i.e., the one or more N-halogenated or N,N-dihalogenated amines and the ammonium salt) can be at a concentration of about 0.02% to about 1%, e.g. about 0.1% to about 0.5%. The ratio for the one or more N-halogenated and N,N-dihalogenated amines to the ammonium salt can be about 1:1. For some applications, the ratio of the one or more N-halogenated or N,N-dihalogenated amines to the ammonium salt can be about 1:0.1.

In another aspect, one or more N-halogenated or N,N-dihalogenated amines may be used for the manufacture of a medicament for treatment or prophylaxis of a bronchopulmonary infection.

For example, one or more N-halogenated or N,N-dihalogenated amines can be used for the manufacture of a medicament for treatment or prophylaxis of a bronchopulmonary infection can result in compositions comprising one or more N-halogenated or N,N-dihalogenated amines at a concentration of about 0.001% to about 10% weight per volume; e.g. about 0.1% to about 5%; e.g. about 0.5% to about 1% active ingredient.

In another aspect, the compositions comprise one or more N-halogenated or N,N-dihalogenated amines in combination with an ammonium salt; e.g. ammonium chloride. For example, NCT and/or NDCT, or their alkylated derivatives can be combined with ammonium chloride. Each of the constituents of the combined composition (i.e., the one or more N-halogenated or N,N-dihalogenated amines and the ammonium salt) can be at a concentration of about 0.02% to about 1%, e.g. about 0.1% to about 0.5%. The ratio of the one or more N-halogenated and N,N-dihalogenated amines to the ammonium salt can be about 1:1. For some applications, the ratio of the one or more N-halogenated or N,N-dihalogenated amines to the ammonium salt can be about 1:0.1.

In one aspect, disclosed is a method for treatment or prophylaxis of a bronchopulmonary infection in a mammal, the method comprises administering to a mammal in need of treatment or prophylaxis a therapeutically effective amount of one or more N-halogenated or N,N-dihalogenated amines. In another aspect, disclosed is a method for treating or preventing obstructive pulmonary disease in a mammal comprising administering to the mammal a therapeutically effective amount of one or more N-halogenated or N,N-dihalogenated amines. In another aspect, disclosed is a method for treating cystic fibrosis in a mammal comprising administering to the mammal a therapeutically effective amount of one or more N-halogenated or N,N-dihalogenated amines. In yet another aspect, disclosed is a method for treating or preventing ventilator-associated infections in a mammal comprising administering to the mammal a therapeutically effective amount of one or more N-halogenated or N,N-dihalogenated amines.

In all of the foregoing methods, the compositions may be administered by inhalation. In certain embodiments, the mammalian subject can be immune-compromised.

It is understood that any aspect, feature, or embodiment of the invention can be combined with any other aspect, feature or embodiment of the invention. Other aspects and advantages of the invention will become apparent from the following description and claims.

### BRIEF DESCRIPTION OF THE FIGURES

Figures not pertaining to the invention are for illustrative purposes only.
FIG. 1 presents arterial partial pressure of oxygen (PaO₂) of pigs during the inhalation period in Example 1 (mean values ± SEM of 6-7 pigs; p = 0.014 between control and 1% NCT + 1% NH₄Cl group; p > 0.05 between all other groups).
FIG. 2 presents pulmonary artery pressure (PAP) of pigs during the inhalation period in Example 4 (mean values ± SEM of 6-7 animals; p < 0.01 for 1% NCT = 1% NH₄Cl and 5% NCT compared to saline and 1% NCT; p > 0.05 between saline and 1% NCT).

### DETAILED DESCRIPTION

The invention is defined by the appended claims.Definitions

As utilized in accordance with the present disclosure, the following terms, unless otherwise indicated, shall be understood to have the following meanings:
"Alkyl" refers to a saturated, branched, or straight-chain monovalent hydrocarbon radical derived by the removal of one hydrogen atom from a single carbon atom of a parent alkane. Alkyl groups include methyl; ethyl; propyls such as propan-1-yl, propan-2-yl (*iso-*propyl), cyclopropan-1-yl, etc.; butyls such as butan-1-yl, butan-2-yl (*sec*-butyl), 2-methyl-propan-1-yl (*iso*-butyl), 2-methyl-propan-2-yl (*t*-butyl), cyclobutan-1-yl; pentyls; hexyls; octyls; dodecyls; octadecyls. An alkyl group comprises from 1 to about 22 carbon atoms, e.g., from 1 to 22 carbon atoms, e.g. from 1 to 12 carbon atoms, or, e.g., from 1 to 6 carbon atoms.

"Alkylcycloalkyl" refers to an alkyl radical, as defined above, attached to a cycloalkyl radical, as defined herein. Alkylcycloalkyl groups includemethyl cyclopentyl, methyl cyclobutyl, ethyl cyclohexyl. An alkylcycloalkyl group comprises from 4 to about 32 carbon atoms, i.e. the alkyl group can comprise from 1 to about 22 carbon atoms and the cycloalkyl group can comprise from 3 to about 10 carbon atoms.

"Active ingredient" refers to a compound of Formulae I, IA, IB, IC, ID, or a salt thereof.

"Acyl" refers to a radical -C(=O)R, where R is hydrogen, alkyl, cycloalkyl, cycloheteroalkyl, aryl, arylalkyl, heteroalkyl, heteroaryl, or heteroarylalkyl as defined herein, each of which may be optionally substituted, as defined herein. Representative examples include formyl, acetyl, cylcohexylcarbonyl, cyclohexylmethylcarbonyl, benzoyl, benzylcarbonyl.

"Acylamino" (or alternatively "acylamido") refers to a radical -NR'C(=O)R, where R' and R are each independently hydrogen, alkyl, cycloalkyl, cycloheteroalkyl, aryl, arylalkyl, heteroalkyl, heteroaryl, or heteroarylalkyl, as defined herein, each of which may be optionally substituted, as defined herein. Representative examples include formylamino, acetylamino (*i.e.,* acetamido), cyclohexylcarbonylamino, cyclohexylmethyl-carbonylamino, benzoylamino (*i.e.,* benzamido), benzylcarbonylamino .

"Acyloxy" refers to a radical -OC(=O)R, where R is hydrogen, alkyl, cycloalkyl, cycloheteroalkyl, aryl, arylalkyl, heteroalkyl, heteroaryl or heteroarylalkyl, as defined herein, each of which may be optionally substituted, as defined herein. Representative examples include acetyloxy (or acetoxy), butanoyloxy, benzoyloxy.

"Alkoxy" refers to a radical -OR where R represents an alkyl or cycloalkyl group as defined herein, each of which may be optionally substituted, as defined herein. Representative examples include methoxy, ethoxy, propoxy, butoxy, cyclohexyloxy.

"Alkoxycarbonyl" refers to a radical -C(=O)-alkoxy where alkoxy is as defined herein.

"Alkylsulfonyl" refers to a radical -S(=O)₂R where R is an alkyl or cycloalkyl group as defined herein, each of which may be optionally substituted, as defined herein. Representative examples include methylsulfonyl, ethylsulfonyl, propylsulfonyl, butylsulfonyl.

"Aryl" refers to an aromatic hydrocarbon group which may be a single aromatic ring or multiple aromatic rings which are fused together, linked covalently, or linked to a common group such as a methylene or ethylene moiety. Aryl groups include groups derived from, acenaphthylene, anthracene, azulene, benzene, biphenyl, chrysene, cyclopentadiene, diphenylmethyl, fluoranthene, fluorene, indane, indene, naphthalene, pentalene, perylene, phenalene, phenanthrene, pyrene, triphenylene. An aryl group comprises from 6 to about 20 carbon atoms, e.g., from 6 to 20 carbon atoms, e.g. from 6 to 10 carbon atoms.

"Arylalkyl" refers to an alkyl group in which one of the hydrogen atoms bonded to a carbon atom is replaced with an aryl group. Arylalkyl groups include benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl. Where specific alkyl moieties are intended, the nomenclature arylalkanyl, arylalkenyl and/or arylalkynyl may be used. An arylalkyl group comprises from 7 to about 42 carbon atoms, e.g. the alkyl group can comprise from 1 to about 22 carbon atoms and the aryl group can comprise from 6 to about 20 carbon atoms.

"Bronchopulmonary infections" refers to infections of both the air passages leading to the lungs, and the lungs themselves and includes, without limitation, obstructive pulmonary disease, cystic fibrosis, and ventilator-associated infections.

"Carbonate" refers to the group CO₃²⁻.

"Carboxylate" refers to the group RCO₂⁻, where R can be hydrogen, alkyl, aryl, cycloalkyl, heteroalkyl, or heteroaryl as defined herein, each of which may be optionally substituted, as defined herein.

"Carbamoyl" refers to the radical -C(=O)N(R)₂ where each R group is independently hydrogen, alkyl, cycloalkyl or aryl as defined herein, which may be optionally substituted, as defined herein.

"Compounds" as used herein refers to any of the compounds encompassed by N-halogenated and N,N-dihalogenated compounds of formulae I, IA, IB, IC, and ID as disclosed herein. These compounds are also referred to herein as haloamines. In one aspect of the current disclosure, haloamines exclude chloramine-T. The compounds may be cationic, or in a salt form. The compounds may be identified either by their chemical structure and/or chemical name. When the chemical structure and chemical name conflict, the chemical structure is determinative of the identity of the compound. The compounds may contain one or more chiral centers and/or double bonds and therefore, may exist as stereoisomers, such as double-bond isomers (*i.e.,* geometric isomers), enantiomers or diastereomers. Accordingly, when stereochemistry at chiral centers is not specified, the chemical structures depicted herein encompass all possible configurations at those chiral centers including the stereoisomerically pure form (*e.g.,* geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the skilled artisan. The compounds may also exist in several tautomeric forms including the enol form, the keto form and mixtures thereof. Accordingly, the chemical structures depicted herein encompass all possible tautomeric forms of the illustrated compounds. Compounds may exist in unsolvated forms as well as solvated forms, including hydrated forms and as N-oxides. In general, the hydrated, solvated and N-oxide forms are within the scope of the present disclosure. Further, it should be understood, when partial structures of the compounds are illustrated, that brackets indicate the point of attachment of the partial structure to the rest of the molecule.

"Cycloalkyl" refers to a saturated cyclic alkyl radical. Typical cycloalkyl groups include groups derived from cyclopropane, cyclobutane, cyclopentane, cyclohexane. A cycloalkyl group comprises from 3 to about 10 carbon atoms, e.g. from 3 to 10 carbon atoms, or, e.g. from 3 to 6 carbon atoms.

"Effective amount" means the amount of a compound that, when administered to a patient for treating or preventing a bronchopulmonary infection or contamination, is sufficient to effect such treatment or prevention. The "effective amount" will vary depending on the compound, the severity of the condition causing the microbial infection and the age, weight, *etc.,* of the patient to be treated.

"Electron-withdrawing group" refers to atoms or functional groups which are electronegative either through a resonance effect or an inductive effect. Examples of such atoms and functional groups include -CO₂R⁰, --NO₂, --SO₃R⁰, --PO₃R⁰R⁰⁰, cyano, halogen (F, Cl, Br, I), and haloalkyl, where R⁰ and R⁰⁰ are independently H, alkyl, aryl, cycloalkyl, heteroalkyl, heteroaryl, or cycloheteroalkyl group, as defined herein, each of which may be optionally substituted.

"Halide" refers to a halogen bearing a negative charge, including fluoride, chloride, bromide, and iodide.

"Halo" refers to a halogen, including fluoro, chloro, bromo and iodo.

"Heteroalkyl" refer to an alkyl radical in which one or more of the carbon atoms (and any associated hydrogen atoms) are each independently replaced with the same or different heteroatomic groups. Heteroatomic groups include --NR⁰--, --O--, --S--, --PH--,-P(O)₂--, --S(O)--, --S(O)₂--, where R⁰ is defined above. Heteroalkyl groups include -O-CH₃, -CH₂-O-CH₃, -S-CH₃, -CH₂-S-CH₃, - NR⁰-CH₃, -CH₂-NR⁰⁰-CH₃, where R⁰ and R⁰⁰ are defined above. A heteroalkyl group can comprise from 1 to about 22 carbon and hetero atoms, e.g., from 1 to 22 carbon and heteroatoms, e.g. from 1 to 12 carbon and hetero atoms, e.g., from 1 to 6 carbon and hetero atoms.

"Heteroaryl" refers to an aryl group in which one or more of the carbon atoms (and any associated hydrogen atoms) are each independently replaced with the same or different heteroatomic groups. Typical heteroatomic groups include --N--, --O--, --S--, and --NR⁰--, where R⁰ is defined above. Typical heteroaryl groups include groups derived from acridine, carbazole, carboline, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene. A heteroaryl group comprises from 5 to about 20 atoms, e.g., from 5 to 20 atoms, e.g. from 5 to 10 atoms.

"Heterocycloalkyl" refers to unsaturated cycloalkyl radical in which one or more carbon atoms (and any associated hydrogen atoms) are independently replaced with the same or different heteroatom. Typical heteroatoms to replace the carbon atom(s) include N, P, O, S, etc. Typical heterocycloalkyl groups include groups derived from epoxides, imidazolidine, morpholine, piperazine, piperidine, pyrazolidine, pyrrolidine, quinuclidine. The heterocycloalkyl group comprises between 3 and 10 carbon atoms.

"Hydroxy" refers to the group OH.

"Microbial" refers to bacteria, fungi (including, e.g., yeast) or virus.

"Phosphate" refers to the group (R)ₙPO₄⁽³⁻ⁿ⁾-, where n is 0, 1 or 2 and R can be hydrogen, alkyl, aryl, cycloalkyl, heteroalkyl, or heteroaryl as defined herein, each of which may be optionally substituted.

"Patient" refers to a mammal (including, *e.g.,* a human), or a bird (including, *e.g.,* a chicken).

"Pharmaceutically acceptable" means that which is useful in preparing a pharmaceutical composition that is generally safe, non-toxic, and neither biologically nor otherwise undesirable, and includes that which is acceptable for veterinary as well as human pharmaceutical use.

"Pharmaceutically acceptable salt" refers to a salt of a compound that is pharmaceutically acceptable and that possesses the desired pharmacological activity of the parent compound. Such salts include acid addition salts formed with inorganic acids such as hydrochloric acid, hydrobromic acid, sulfuric acid, nitric acid, phosphoric acid; or formed with organic acids such as acetic acid, benzenesulfonic acid, benzoic acid, camphorsulfonic acid, citric acid, ethanesulfonic acid, fumaric acid, glucoheptonic acid, gluconic acid, lactic acid, maleic acid, malonic acid, mandelic acid, methanesulfonic acid, 2-napththalenesulfonic acid, oleic acid, palmitic acid, propionic acid, stearic acid, succinic acid, tartaric acid, p-toluenesulfonic acid, trimethylacetic acid and salts formed when an acidic proton present in the parent compound is replaced by either a metal ion, *e.g.,* an alkali metal ion, an alkaline earth ion, or an aluminum ion; or coordinates with an organic base such as diethanolamine, triethanolamine, N-methylglucamine. A representative list of pharmaceutically acceptable salts can be found in S.M. Berge et al., J. Pharma Sci., 66(1), 1-19 (1977), and Remington: The Science and Practice of Pharmacy, R. Hendrickson, ed., 21st edition, Lippincott, Williams & Wilkins, Philadelphia, PA, (2005), at p. 732, Table 38-5.

"Pharmaceutically acceptable carrier" refers to a pharmaceutically acceptable diluent, adjuvant, excipient or vehicle with which a compound is combined for administration.

"Pharmaceutical composition" as used herein comprises one or more compounds of formulae I, IA, IB, IC or ID, specific compounds encompassed by these formula, and a pharmaceutically acceptable carrier.

"Prevent", "preventing" and "prevention" of a microbial infection refer to reducing the risk of a patient from developing a microbial infection, or reducing the frequency or severity of a microbial infection in a patient.

"Salt" refers to a cation or anion (e.g. a cationic or anionic compound of formulae I, IA, IB, IC and ID) coupled with an anion or a cation, either in solution or as a solid. Salts include pharmaceutically acceptable salts as well as solvent addition forms (solvates) of the same salt. Unless specified in reaction schemes, where certain compounds described herein are named or depicted as a particular salt (e.g. the chloride), all other salt forms are within the scope of this disclosure.

"Sulfate" refers to the group ⁻OSO₃H or SO₄²⁻.

"Sulfonate" refers to the group ⁻OSO₂R, where R can be alkyl, aryl, cycloalkyl, heteroalkyl or heteroaryl.

A "substituted" group refers to a group wherein from 1 to about 5 (e.g. from 1 to 5, e.g. from 1 to 3) hydrogens are replaced with a substituent such as an acyl, alkoxy, alkyl, alkoxycarbonyl, alkylsulfonyl" amino, acyloxy, aryl, carboxyl, carbamoyl, cycloalkyl, halo, heteroalkyl, heteroaryl, cycloheteroaryl, oxo, hydroxy, acylamino, electron-withdrawing group, or a combination thereof. In certain aspects, substituents include, without limitation, cyano, hydroxy, nitro, trifluoromethyl, methoxy, phenyl, and carboxyl.

### Compounds

The current disclosure relates to N-halogenated and N,N-dihalogenated amines and their use in the treatment and prophylaxis of bronchopulmonary infections and other conditions in mammals.

One embodiment of the current disclosure relates to N-halogenated and N,N-dihalogenated compounds of formula (I):

A-C(R¹R²)R(CH₂)ₙC(R³R⁴)-Y-Z (I)

or a derivative thereof, said derivative being selected from the group consisting of pharmaceutically acceptable salts, esters, and amides with (C₁-C₆)alkanols, wherein
A is hydrogen, HalNH- or Hal₂N-;
Hal is a halogen selected from the group consisting of chloro, bromo and iodo;
R¹ is hydrogen or an optionally substituted group selected from the group consisting of alkyl, cycloalkyl, heteroalkyl, aryl, heteroaryl, and heterocycloalkyl groups, and -COOH;
R² is hydrogen or an optionally substituted group selected from the group consisting of alkyl, cycloalkyl, heteroalkyl, aryl, heteroaryl, and heterocycloalkyl groups, or R¹ and R² together with the carbon atom to which they attach form an optionally substituted cycloalkyl or heterocycloalkyl group;
R is a carbon-carbon single bond or a divalent cycloalkylene radical with three to six carbon atoms,
n is 0 or an integer from 1 to 13;
R³ and R⁴ are each independently selected from the group consisting of hydrogen, fluoro, -NHHal, NHal₂, and an optionally substituted group selected from the group consisting of alkyl, cycloalkyl, heteroalkyl, aryl, heteroaryl, and heterocycloalkyl groups;
Y is selected from a group consisting of
   a single bond; -O-; a divalent (C₁₋₁₈)alkyl group in which, optionally, one or two methylene groups are replaced with a mono- or di-substituted methylene group; and a divalent (C₁₋₁₈)heteroalkyl group wherein the divalent (C₁₋₁₈)heteroalkyl group is a divalent (C₁₋₁₈)alkyl group in which, optionally, one or two methylene groups are replaced with 1 or 2 -NR'-, -O-, -S-, -S(=O)-, >C=O, -C(=O)O-, -OC(=O)-, -C(=O)NH-, -NHC(=O)-, -C(=O)NR'-, -NR'C(=O)-, -S(=O)₂-, -S(=O)₂NR'-, -S(=O)₂NH-, -NR'S(=O)₂- or -NHS(=O)₂-;
R' is selected from the group consisting of hydrogen, Cl, Br, (C₁₋₅)alkyl, (C₃₋₆)cycloalkyl, (C₆₋₁₀)aryl, (C₆₋₁₀)aryl(C₁₋₄)alkyl, (C₁₋₅)alkylNHC(=O)-, (C₁₋₅)alkoxyC(=O)-, R^{a}R^{b}NC(=O)-, (C₁₋₅)alkylC(=O)-, (C₆₋₁₀)arylC(=O)-, (C₆₋₁₀)aryl(C₁₋₄)alkylC(=O)-, (C₆₋₁₄)aryl, heteroaryl comprising 4 to 10 ring atoms with at least one heteroatom selected from O, S and N in the ring, and heterocycloalkyl containing 2-10 carbon atoms and 1 to 4 heteroatoms selected from N, O or S;
R^{a} and R^{b} are each independently hydrogen, (C₁₋₅)alkyl, (C₃₋₆)cycloalkyl, (C₁₋₅)alkylNHC(=O)-, (C₁₋₅)alkylC(=O)-, (C₆₋₁₄)aryl, (C₆₋₁₀)aryl(C₁₋₄)alkyl, heteroaryl comprising 4 to 10 ring atoms with at least one heteroatom selected from O, S and N in the ring, or heterocycloalkyl(C₁₋₄) alkyl, the heterocycloalkyl group containing 2-10 carbon atoms and 1 to 4 heteroatoms selected from N, O or S;
Z is selected from the group consisting of hydrogen, -SO₃H, -SO₂NH₂,-P(=O)(OH)₂, -B(OH)₂, -[X(R⁵)(R⁶)R⁷]Q, -S(=O)₂NR^{c}R^{d}, -S(=O)₂NHC(=O)R^{e}, S(=O)₂OC(=O)NR^{c}R^{d}, -S(=O)₂NR^{c}C(=O)NR^{c}R^{d} and -S(=O)₂(N=)C(OH)NR^{c}R^{d} wherein R^{c} and R^{d} are each independently hydrogen or is independently selected from the group consisting of (C₁₋₅)alkyl, (C₃₋₆)cycloalkyl, (C₁₋₅)alkylNHC(=O)-, (C₁₋₅)alkylC(=O)-, (C₆₋₁₀)arylC(=O)-, (C₆₋₁₀)aryl(C₁₋₄)C(=O)-, (C₆₋₁₄)aryl, (C₆₋₁₀)arly(C₁₋₄)alkyl, heteroaryl comprising 4 to 10 ring atoms with at least one heteroatom selected from O, S and N in the ring, and heterocycloalkyl containing 2-10 carbon atoms and 1 to 4 heteroatoms selected from N, O or S, and R^{e} is hydrogen or is selected from the group consisting of (C₁₋₅)alkyl, (C₃₋₆)cycloalkyl, (C₆₋₁₄)aryl, (C₆₋₁₀)aryl(C₁₋₄)alkyl, heteroaryl comprising 4 to 10 ring atoms with at least one heteroatom selected from O, S and N in the ring, and heterocycloalkyl containing 2-10 carbon atoms and 1 to 4 heteroatoms selected from N, O or S; or a salt, an amine oxide thereof, or a derivative or a bioisostere or a prodrug thereof;
X is selected from the group consisting of N, P, and S;
Q is a counter anion or is absent;
R⁵ and R⁶ are each independently selected from the group consisting of alkyl, aryl, cycloalkyl, heteroalkyl, heteroaryl, and heterocycloalkyl, each of which may be optionally substituted; or R⁵ and R⁶ together with the X atom to which they are attached form heterocycloalkyl group, each of which may be optionally substituted; and
R⁷ is alkyl, aryl, cycloalkyl, heteroalkyl, heteroaryl, or heterocycloalkyl, each of which may be optionally substituted, and may further be O when X is N, with the proviso that R⁷ is absent when X is S; and with the proviso that if R is a divalent cycloalkylene radical, n will not exceed the integer 11.

One aspect of the current disclosure relates to N-halogenated and N,N-dihalogenated compounds of formula (IA)

A-C(R¹R²)R(CH₂)ₙC(R³R⁴)-Y-Z (IA)

or a derivative thereof, said derivative being selected from the group consisting of pharmaceutically acceptable salts, esters, and amides with (C₁₋₆)alkanols, wherein
A is hydrogen, Hal₂N-, or HalHN;
Hal is halogen selected from the group consisting of chloro, bromo and iodo;
R¹ is hydrogen, (C₁₋₆)alkyl or the group -COOH;
R² is hydrogen or (C₁₋₆)alkyl, or R¹ and R² together with the carbon atom to which they attach form a (C₃₋₆)cycloalkyl ring;
R is a carbon-carbon single bond or a divalent cycloalkylene radical with three to six carbon atoms;
n is 0 to 13,
R³ is hydrogen, (C₁₋₆)alkyl, -NHHal, or NHal₂;
R⁴ is hydrogen or (C₁₋₆)alkyl;
Y is a single bond;
and Z is selected from the group consisting of hydrogen, -SO₃H, -SO₂NH₂,-P(=O)(OH)₂ and -B(OH)₂.

Within this aspect, if R is a divalent cycloalkylene radical n will not exceed the integer 11. That is, n may be 0, 1, 2, 3, 4, 5, 6, 7, 8, 9, 10, or 11. In other words, an amino acid including an Z acidic group will have up to 16 chain atoms. In the divalent cycloalkylene radical or in the divalent radical -(CH₂)n- one hydrogen may be substituted with NHal₂. Compounds of formula (IA) may contain up to a total of three NHal₂ or -NHHal groups, for example, one or two -NHal₂ or -NHHal groups. In certain aspects, compounds of formula (IA) contain one -NHal₂ group, which may be in the alpha-, beta-, gamma-, delta-, epsilon-, or omega- position of an acidic R¹ (if R¹ is -COOH) or Z group.

Another aspect of the current disclosure relates to N-halogenated and N,N-dihalogenated compounds of formula (IB)

A-C(R¹R²)-C(R³R⁴)-Y-Z (IB)

or derivative thereof, said derivative being selected from the group consisting of pharmaceutically acceptable salts, esters, and amides with (C₁₋₆)alkanols, wherein
A is selected from the group consisting of hydrogen, Hal₂N-, and HalHN;
Hal is halogen selected from the group consisting of chloro and bromo;
R¹ and R² are each independently selected from the group consisting of (C₁₋₅)alkyl, heteroalkyl, halo(C₁₋₅)alkyl, (C₃₋₆)cycloalkyl, (C₃₋₆)cycloalkyl(C₁₋₃)alkyl, (C₆-₁₀)aryl(C₁₋₄)alkyl, (C₆₋₁₄)aryl, heteroaryl, and (C₃₋₁₀)heterocycloalkyl, or R₁ and R₂ together with the carbon atom to which they are attached to form a (C₃₋₁₂) cycloalkyl or (C₃₋₁₂)heterocycloalkyl;
R³ and R⁴ are each independently selected from the group consisting of hydrogen, fluoro, (C₁₋₅)alkyl, heteroalkyl, halo(C₁₋₅)alkyl, (C₃₋₆)cycloalkyl, (C₃-₆)cycloalkyl(C₁₋₃)alkyl, (C₆₋₁₀)aryyl(C₁₋₄)alkyl, (C₆₋₁₄)aryl, heteroaryl, and (C₃₋₁₀) heterocycloalkyl, or R₁ and R₂ together with the carbon atom to which they are attached to form a (C₃₋₁₂) cycloalkyl, or (C₃₋₁₂) heterocycloalkyl;
Y is selected from a group consisting of single bond, -O-, a divalent (C₁₋₁₈)alkyl group in which optionally one or two methylene groups are replaced with a mono- or di-substituted methylene group, and a (C₁₋₁₈)heteroalkyl group;
with the proviso that when R¹ is (C₁₋₅)alkyl or when R¹ and R² together with the carbon atom to which they attach form a (C₃₋₆)cycloalkyl, then Y must be -O- or a divalent (C₁₋₁₈) alkyl group wherein one or two methylene groups are replaced with a substituted methylene group or Y must be a divalent (C₁₋₁₈) heteroalkyl group wherein the (C₁₋₁₈) heteroalkyl group is a (C₁₋₁₈)alkyl group where one or two methylene groups are replaced with a by -NR'-, -O-, -S-, -S(=O)- or -S(=O)₂-;
R' is hydrogen or is selected from the group consisting of Cl, Br, (C₁₋₅)alkyl, (C₃₋6)cycloalkyl, (C₆₋₁₀)aryl, (C₆₋₁₀)aryl(C₁₋₄)alkyl, (C₁₋₅)alkylNHC(=O)-, (C₁₋₅)alkoxyC(=O)-, R^{a}R^{b}NC(=O)-, (C₁₋₅)alkylC(=O)-, (C₆₋₁₀)arylC(=O)-, (C₆₋₁₀)aryl(C₁₋₄)alkylC(=O)-, (C₆₋₁₄)aryl, heteroaryl comprising 4 to 10 ring atoms with at least one heteroatom selected from O, S and N in the ring, and heterocycloalkyl containing 2-10 carbon atoms and 1 to 4 heteroatoms selected from N, O or S;
R^{a} and R^{b} are each independently hydrogen, (C₁₋₅)alkyl, (C₃₋₆)cycloalkyl, (C₁₋₅)alkylNHC(=O)-, (C₁₋₅)alkylC(=O)-, (C₆₋₁₄)aryl, (C₆₋₁₀)aryl(C₁₋₄)alkyl, heteroaryl comprising 4 to 10 ring atoms with at least one heteroatom selected from O, S and N in the ring, or heterocycloalkyl(C₁₋₄) alkyl, the heterocycloalkyl group containing 2-10 carbon atoms and 1 to 4 heteroatoms selected from N, O or S; and
Z is selected from the group consisting of -SO₃H, -SO₂NH₂, -P(=O)(OH)₂, a salt or ester thereof, and an acid isostere thereof but not -C(=O)OH.

Another aspect of the current disclosure relates to compounds of formula (IB), wherein R¹ and R² together with the carbon atoms to which they are attached form a ring system with 4 to 7 carbon ring members, wherein optionally one or two ring members are nitrogen.

Another aspect of the current disclosure relates to N-halogenated and N,N-dihalogenated compounds of formula (IC)

A-C(R¹R²)(CH₂)ₙY(CH₂)ₘ-Z (IC)

or a derivative thereof, said derivative being selected from the group consisting of pharmaceutically acceptable salts, esters, and amides with (C₁-C₆)alkanols, wherein:
A is HalHN- or Hal₂N-;
Hal is halogen selected from the group consisting of chloro and bromo;
R¹ and R² are each independently selected from the group consisting of alkyl, aryl, cycloalkyl, heteroalkyl, heteroaryl, and heterocycloalkyl, each of which may be optionally substituted; or R¹ and R² together with the carbon atom to which they are attached form a cycloalkyl or heterocycloalkyl group, each of which may be optionally substituted;
Y is selected from the group consisting of a single bond, --O--, --CF₂--, --CHF--,-C(=O)--, --C(=O)O--, --OC(=O)--, --C(=O)NR^{a}--, --NR^{a}C(=O)--, --P(=O)(OR^{b})O--, -OP(=O)(OR^{b})-, --P(=O)(OR^{b})NR^{c}--, --NR^{c}P(=O)(OR^{b})--, --S(=O)₂,-S(=O)₂O--,--OS(=O)₂--, --S(=O)₂NR^{d}--, --NR^{d}S(=O)₂--, or heteroaryl;
R^{a}, R^{b}, R^{c} and R^{d} are each independently selected from the group consisting of hydrogen, and optionally substituted alkyl, aryl, cycloalkyl, heteroalkyl, heteroaryl, and heterocycloalkyl;
Z is -[X(R⁵)(R⁶)R⁷]Q,
X is N, P, or S;
Q is a counter ion or absent;
R⁵, R⁶, and R⁷ are defined as in formula (I) above;
n is 0 or is an integer from 1 to 12; and m is an integer from 1 to 12.

Another aspect of the current disclosure relates to the following N-halogenated and N,N-dihalogenated compounds of formula (ID) or a derivative thereof; the derivative being selected from the group consisting of pharmaceutically acceptable salts and esters with (C₁₋C₆)alkanols:

A-C(R₁R₂)(CH₂)ₙC(R³R⁴)-Z (ID)

or a derivative thereof, said derivative being selected from the group consisting of pharmaceutically acceptable salts, esters, and amides with (C₁₋₆)alkanols, wherein:
A is hydrogen, HalNH- or Hal₂N-;
Hal is halogen selected from the group consisting of chloro, bromo and iodo;
R¹ and R² are each independently selected from an optionally substituted group selected from the group consisting of alkyl, cycloalkyl, heteroalkyl, heteroaryl, and heterocycloalkyl groups, or R¹ and R² together with the carbon atom to which they attach form a (C₃₋₆)cycloalkyl ring.
R³ and R⁴ are independently selected from the group consisting of hydrogen, fluoro, and an optionally substituted group selected from the group consisting of alkyl, cycloalkyl, heteroalkyl, heteroaryl, and heterocycloalkyl groups;
Z is selected from the group consisting of, -SO₃H, -SO₂NH₂, -P(=O)(OH)₂,-B(OH)₂, and -[X(R⁵)(R⁶)R⁷]Q;
X is selected from the group consisting of N, P, and S;
Q is a counter anion or is absent;
R⁵ and R⁶ are each independently selected from the group consisting of alkyl, aryl, cycloalkyl, heteroalkyl, heteroaryl, and heterocycloalkyl, each of which may be optionally substituted; or R⁵ and R⁶ together with the X atom to which they are attached form heterocycloalkyl group, each of which may be optionally substituted; and
R⁷ is alkyl, aryl, cycloalkyl, heteroalkyl, heteroaryl, or heterocycloalkyl, each of which may be optionally substituted, and may further be O when X is N, with the proviso that R⁷ is absent when X is S; and n is 0 or an integer from 1 to 6.

In another aspects, the above-described compositions include the following compounds or a derivative thereof; said derivative being selected from the group consisting of pharmaceutically acceptable salts, esters, and amides with (C₁-C₆)alkanols:
N,N-dichlorotaurine;
N,N-dichloro-2-methyltaurine;
N,N-dichloro-2,2,3,3-tetramethyl-ß-alanine;
N,N-dichloro-2,2-dimethyltaurine;
N,N-dichloro-1,1,2,2-tetramethyltaurine;
N,N-dibromo-2,2-dimethyltaurine;
N,N-dibromo-1,1,2,2-tetramethyltaurine;
N,N-diiodotaurine;
N,N-dichloro-3,3-dimethylhomotaurine;
N,N-dichloro-2-methyl-2-amino-ethanesulfonic acid; and
N,N-dichloro-1-methyl-ethanesulfonic acid,
N,N-dichloro amino-trimethylene phosphonic acid;
N,N-dibromo-2-amino-5-phosphonopantanoic acid;
N,N-dichloro amino-ethylphosponic acid diesters, such as the diethylester;
N,N-dichloro-1-amino-1-methylethane phosphonic acid;
N,N-dichloro-1-amino-2-methylethane phosphonic acid;
N,N-dichloro-1-amino-2-methylpropane phosphonic acid;
N,N-dichloro-leucine phosphonic acid;
N,N-dichloro-4-amino-4-phosphonobutyric acid;
(±) N,N-dichloro-2-amino-5-phosphonovaleric acid;
N,N-dichloro-(+)2-amino-5-phosphonovaleric acid;
N,N-dichloro d,1-2-amino-3-phosphonopropionic acid;
N,N-dichloro-2-amino-8-phosphonooctanoic acid;
N,N-dichloro-leucine boronic acid;
N,N-dichloro-β-alanine boronic acid;
N-chlorotaurine;
N-chloro-2-methyltaurine;
N-chloro-2,2,3,3-tetramethyl-ß-alanine;
N-chloro-2,2-dimethyltaurine;
N-chloro-1,1,2,2-tetramethyltaurine;
N-bromo-2,2-dimethyltaurine;
N-bromo-1,1,2,2-tetramethyltaurine;
N-iodotaurine;
N-chloro-3,3-dimethylhomotaurine;
N-chloro-2-methyl-2-amino-ethanesulfonic acid; and
N-chloro-1-methyl-ethanesulfonic acid,
N-chloro amino-trimethylene phosphonic acid;
N-bromo-2-amino-5-phosphonopantanoic acid;
N-chloro amino-ethylphosponic acid diesters, such as the diethylester;
N-chloro-1-amino-1-methylethane phosphonic acid;
N-chloro-1-amino-2-methylethane phosphonic acid;
N-chloro-1-amino-2-methylpropane phosphonic acid;
N-chloro-leucine phosphonic acid;
N-chloro-4-amino-4-phosphonobutyric acid;
(±) N-chloro-2-amino-5-phosphonovaleric acid;
N-chloro-(+)2-amino-5-phosphonovaleric acid;
N-chloro d,1-2-amino-3-phosphonopropionic acid;
N-chloro-2-amino-8-phosphonooctanoic acid;
N-chloro-leucine boronic acid;
N-chloro-β-alanine boronic acid;
(1-(dichloroamino)cyclohexyl)methanesulfonic acid;
(1-(chloroamino)cyclohexyl)methanesulfonic acid;
2-(chloroamino)-N,N,N-2-tetramethylpropan-1-ammonium chloride;
2-(dichloroamino)-N,N,N-2-tetramethylpropan-1-ammonium chloride;
3-(chloroamino)-N,N,N-3-tetramethylbutan-1-ammonium chloride;
3-(dichloroamino)-N,N,N-3-tetramethylbutan-1-ammonium chloride;
1-(2-(dichloroamino)-2-methylpropyl)-1-methylpiperidinium chloride;
1-(2-(chloroamino)-2-methylpropyl)-1-methylpiperidinium chloride;
(2-(dichloroamino)-2-methylpropyl)dimethylsulfonium chloride;
(2-(chloroamino)-2-methylpropyl)dimethylsulfonium chloride;
(4-(dichloroamino)-4-methylpentyl)trimethylphosphonium chloride;
(4-(chloroamino)-4-methylpentyl)trimethylphosphonium chloride;
3-(3-(dichloroamino)-3-methylbutylsulfonyl)-N,N,N-trimethylpropan-1-aminium chloride;;
3-(3-(chloroamino)-3-methylbutylsulfonyl)-N,N,N-trimethylpropan-1-aminium chloride;
2-(3-(dichloroamino)-3-methylbutylsulfonyl)-N,N,N-trimethylethanaminium chloride;
2-(3-(chloroamino)-3-methylbutylsulfonyl)-N,N,N-trimethylethanaminium chloride.

In another aspect, the compounds of the current disclosure relate to HalHN- or Hal₂N- derivatives selected from the group consisting of (e.g., glycine, alanine, leucine), a β-amino carbonic acid (e.g., β-alanine,), and a-amino sulfonic acid (e.g., aminomethane sulfonic acid), a β-amino sulfonic acid (e.g., taurine and its derivatives alkylated at a carbon, e.g., dimethyltaurine), and an aliphatic amine (e.g., ethylamine). In certain aspects, the N-halogenated or N,N-dihalogenated amine is N-chlorotaurine (NCT) or N,N-dichlorotaurine (NDCT), or pharmaceutically acceptable salts or esters thereof. In another aspect, the N-halogenated amine is NCT.

The present disclosure provides one or more N-halogenated and N,N-dihalogenated amines in the treatment and prophylaxis of bronchopulmonary infections and other conditions in mammals. As used herein, the terms "N-halogenated amine(s)" and "N,N-dihalogenated amine(s)" include analogues and derivatives thereof, and pharmaceutically acceptable salts or esters of any of the foregoing compounds. Suitable salts can be prepared by known methods, including the method described in German Patent Application 4041703 by Gottardi. Suitable salts include sodium and potassium salts.

The halogen of the N-halogenated and N,N-dihalogenated amines may be any group 17 element, such as fluorine, chlorine bromine, or iodine.

The amine of the N-halogenated and N,N-dihalogenated amines may be any amines including, e.g., a protein, peptide, or amino acid. Additionally, the amine can be derived from at least one of an α-amino carbonic acid (e.g., glycine, alanine, leucine), a β-amino carbonic acid (e.g., β-alanine), an α-amino sulfonic acid (e.g., aminomethane sulfonic acid), a β-amino sulfonic acid (e.g., taurine and its derivatives alkylated at a carbon, e.g., dimethyltaurine), and an aliphatic amine (e.g., ethylamine). The amine can be taurine. In certain embodiments, the one or more N-halogenated or N,N-dihalogenated amines can be N-chlorotaurine (NCT) or N,N-dichlorotaurine (NDCT), or pharmaceutically acceptable salts or esters thereof. For example, the N-halogenated amine can be NCT or its sodium salt.

Applicant refers to U.S. provisional applications 60/989,274 (filed 11/20/2007), 60/992,167 (filed 12/4/2007), and 61/028,682 (filed 02/14/2008).

### Compositions and formulations

The N-halogenated and N,N-dihalogenated amines can be combined with an ammonium salt; e.g. ammonium chloride. This combination can lead to formation of monochloramine in equilibrium which is lipophilic and penetrates pathogens better than the haloamine alone [Refs. 4,8].

N-halogenated and N,N-dihalogenated amines can be synthesized by known techniques. For example, NCT can be synthesized as a crystalline sodium salt in aqueous solution [Ref. 3]. Similarly, NDCT can be synthesized as described in Refs. 16 and 17.

Formulations of NCT and ammonium chloride may be synthesized as disclosed in Ref. 18.

In various embodiments, the compositions can include pharmaceutically acceptable carriers, such as buffers, stabilizers, solvents, preserving agents, diluents, extenders and other recognized auxiliary substances or excipients. The one or more N-halogenated and N,N-dihalogenated amines can be present in compositions at a concentration of about 0.001 % to about 10% weight per volume, e.g. about 0.1 % to about 5%, e.g. about 0.5% to about 1%.

The compositions and methods of the disclosure may include one or more N-halogenated or N,N-dihalogenated amines in combination with an ammonium salt, e.g. ammonium chloride. One example is a combination of NCT and/or NDCT (or their derivatives), with ammonium chloride. Each of the constituents of the combined composition (i.e., the one or more N-halogenated or N,N-dihalogenated amines and the ammonium salt) can be at a concentration of about 0.02% to about 1%, e.g. about 0.1 % to about 0.5%. The ratio for the one or more N-halogenated and N,N-dihalogenated amines to the ammonium salt can be about 1: 1. For some applications, the ratio of the one or more N-halogenated or N,N-dihalogenated amines to the ammonium salt can be about 1:0.1.

### Treatment methods

Methods of using compositions comprising one or more of the N-halogenated or N,N-dihalogenated amines include treatment or prophylaxis of bronchitis, pneumonia, other bronchopulmonary infections caused by viruses, bacteria, fungi, protozoa, spores and/or worms, obstructive pulmonary disease (COPD), cystic fibrosis, and ventilator associated infections. The compositions can be administered to a mammalian subject according to known methods. For bronchopulmonary applications, the compositions can be administration by inhalation or by bronchial lavage.

One exemplary N-halogenated amine is NCT, the N-chloro derivative of the amino acid taurine, which is a long-lived oxidant produced by human leukocytes during inflammation [Ref. 19]. NCT can save the oxidation capacity of originally formed hypochlorous acid, which can be detoxified by reaction with taurine (HOCI + taurine -N-chlorotaurine + H₂O) [Ref. 20]. In addition, NCT can down-regulate proinflammatory cytokines and therefore it may be involved in termination of inflammation [Refs. 21-23].

The disclosure, in all of its various aspects and embodiments, comprises one or more N-halogenated and N,N-dihalogenated amines in the treatment and prophylaxis of bronchopulmonary infections and other conditions in mammals. As used herein, the terms "N-halogenated amine(s)" and "N,N-dihalogenated amine(s)" include analogues and derivatives thereof, and pharmaceutically acceptable salts or esters of any of the foregoing compounds. Derivatives include alkylated derivatives such as N,N-dichloro-2,2-dimethyl taurine. Suitable salts can be prepared by known methods, including the method described in German Patent Application 4041703 by Gottardi. Suitable salts include sodium and potassium salts.

The halogen of the N-halogenated and N,N-dihalogenated amines may be any group 17 element, such as fluorine, chlorine, bromine, or iodine. In various embodiments, an N-halogenated or N,N-dihalogenated amine can be a derivative of a protein, peptide, or amino acid, or pharmaceutically acceptable salts thereof. Additionally, the amine can be derived from at least one of an α-amino carbonic acid (also referred to herein as α-amino acid or α-amino carboxylic acid, such as glycine, alanine, leucine), a β-amino carbonic acid (e.g., β-alanine), an α-amino sulfonic acid (e.g., aminomethane sulfonic acid), a β-amino sulfonic acid (e.g., taurine and its derivatives alkylated at a carbon, e.g., dimethyltaurine), and an aliphatic amine (e.g., ethylamine). In certain embodiments, the N-halogenated or N,N-dihalogenated amine can be N-chlorotaurine (NCT) or N,N-dichlorotaurine (NDCT), or a mixture thereof, or pharmaceutically acceptable salts or esters thereof, e.g. an alkali salt, such as the sodium salt.

In certain embodiments of the invention, the one or more N-halogenated or N,N-dihalogenated amines are N-chlorotaurine (NCT) or N,N-dichlorotaurine (NDCT), or pharmaceutically acceptable salts or esters thereof.

In certain embodiments, compositions and methods using one or more N-halogenated and N,N-dihalogenated amines can also comprise an ammonium salt; e.g. ammonium chloride. The addition of an ammonium salt to composition of N-halogenated and N,N-dihalogenated amines can lead to formation of monochloramine in equilibrium which is lipophilic and penetrates pathogens better than the haloamine alone [Refs. 4, 8].

N-halogenated and N,N-dihalogenated amines can be synthesized by known techniques. For example, NCT can be synthesized as a crystalline sodium salt in aqueous solution [Ref. 3]. Similarly, NDCT can be synthesized as described in Refs. 16 and 17.

Formulations of NCT and ammonium chloride may be synthesized as disclosed in Ref. 18.

In various embodiments, the compositions can include pharmaceutically acceptable carriers, such as buffers, stabilizers, solvents, preserving agents, diluents, extenders and other recognized auxiliary substances or excipients.

In certain embodiments, the one or more N-halogenated and N,N-dihalogenated amines are present or administered at a concentration of about 0.001 % to about 10% weight per volume; e.g. about 0.1 % to about 5%, e.g. about 0.5% to about 1%. In embodiments, the ratio of the one or more N-halogenated and N,N-dihalogenated amines to the ammonium salt can be about 1: 1. For some applications, the ratio of the one or more N-halogenated or N,N-dihalogenated amines to the ammonium salt can be about 1:0.1.

Also described herein are methods for the treatment or prophylaxis of bronchitis, pneumonia, other bronchopulmonary infections caused by viruses, bacteria, fungi, protozoa, spores and/or worms, obstructive pulmonary disease (COPD), cystic fibrosis, and ventilator-associated infections.

The compositions described herein can be administered to a mammalian subject according to known methods. For instance, the compositions may be administered by inhalation, bronchial lavage, nasal or buccal administration. Other administration methods known to those of skill in the art may be used.

One exemplary N-halogenated amine is NCT, the N-chloro derivative of the amino acid taurine, which is a long-lived oxidant produced by human leukocytes during inflammation [Ref. 19]. NCT can save the oxidation capacity of originally formed hypochlorous acid, which can be detoxified by reaction with taurine (HOCI + taurine -N-chlorotaurine + H₂0) [Ref. 20]. In addition, NCT can down-regulate pro-inflammatory cytokines and therefore it may be involved in termination of inflammation [Refs. 21-23].

NCT is relatively safe and well-tolerated by mammals. For example, as a mild oxidant, tolerability of 1% aqueous NCT solution by human and animal tissue is good. This was demonstrated in rabbit and human eyes [Ref. 26]. Also, data indicating efficacy in infectious conjunctivitis are available [Ref. 27]. In human external otitis, NCT was more effective than a standard medication [Ref. 28]. A pilot study in chronic rhinosinusitis demonstrated good tolerability [Ref. 29]. Treatment of purulent coated crural ulcers with NCT caused significantly less pain and was less toxic than chloramine T, the standard for decades in our University hospital [Ref. 30]. It is possible to eradicate bacteria from the urinary bladder with NCT irrigations as shown in three patients suffering from inflammation with omniresistant Pseudomonas aeruginosa [Ref. 31]. Furthermore, transtympanal injection of 0.1 %, 1%, and 10% NCT to the middle ear of mice did not cause damage of the inner ear [Ref. 32]. The same was true for guinea pigs where 10 µL of 1% and 0.1% were injected repeatedly to the middle ear via an implanted catheter system [Ref. 33]. Local administration of NCT inhibited septic arthritis by Staphylococcus aureus in a mouse model [Ref. 34]. In a further study, Swiss mice tolerated up to one mL 1% NCT upon intraperitoneal injection.

NCT and other N-chloramines have broad-spectrum antimicrobial activity including representatives of all classes of pathogens [Refs. 4-7 and 24] and may contribute to inactivation of pathogens *in vivo* [Ref. 26]. Because of the unspecific oxidizing mechanism of reaction, resistance of pathogens is not induced by treatment with NCT [Ref. 4]. See also Ref. 35.

Furthermore, certain natural N-chloramines do not decompose to toxic compounds (above all NCT and other N-haloamines), and no signs for systemic resorption have been observed in the above mentioned clinical studies. N-chloramines react with reducing agents according to R₂N-Cl + H⁺ + 2e⁻ → R₂-NH + Cl- wherein each R is independently moieties as defined in formulae (IA) -(ID) (e.g., NCT converts to the endogenous products taurine and chloride by the reaction: ClHN-CH₂-CH₂-SO₃⁻ + 2H⁺ + 2e⁻ → H₃N⁺-CH₂-CH₂-SO₃⁻ + Cl-). The absence of residues and decay products can be a general advantage of haloamines compared to other antimicrobial agents.

### Tolerability of inhaled haloamines

Despite the fact that haloamines, for instance chloramine-T (N-chloro toluolsulfonamide), are known to cause irritative and toxic effects when inhaled [Refs. 1 and 2], we found that NCT is well tolerated in the bronchopulmonary system upon inhalation. A study was designed to evaluate the tolerability of inhalative haloamines in a pig model. NCT was used as a representative haloamine. Anesthetized pigs inhaled test solutions of 1% NCT (n = 7), 5% NCT (n = 6), or 1% NCT plus 1% ammonium chloride (n = 6), and 0.9% saline solution as a control (n = 7), respectively. Applications were performed every hour over a four-hour time period, i.e., four inhalations in total, with 5 mL each. One hour after the last dosing, the animals were euthanized. Oxygenation parameters of animals treated with 1% NCT were similar to those of the controls treated with saline. Surprisingly, some parameters (alveolo-arterial difference of oxygen partial pressure, pulmonary artery pressure) tended to result in even better values in the 1% NCT group than in the saline group. Histological investigations revealed no statistical difference between the test groups and the saline group. Addition of ammonium chloride in the applied high concentration of 1% to 1% NCT provoked statistically significant impact on blood oxygenation, which would require adjustment of dose. 5% NCT was very well tolerated also; only the pulmonary artery pressure became higher than in the control group.

In addition, three human volunteers suffering from viral bronchitis inhaled 5 mL of 1% NCT for 10 minutes 3 times daily for three days. There were no side effects detectable, particularly no symptoms for bronchoconstriction, such as cough or dyspnea. Indeed, patients experienced easier breathing, less coughing and less expectoration. One patient who usually developed bronchitis after pharyngitis did not develop bronchitis when he used NCT as a treatment for pharyngitis.

These results clearly show that 1% NCT is very well tolerated in the bronchopulmonary system upon inhalation and can be applied to treat bronchopulmonary infections, such as bronchitis and pneumonia. Particularly, patients suffering from chronic obstructive pulmonary disease (COPD) who experience repeated bronchopulmonary infections would strongly profit by NCT inhalations.

### Bronchopulmonary applications for haloamines

One exemplary bronchopulmonary application for haloamines is the treatment of patients suffering from cystic fibrosis. Because of an inborn defect of an ion channel, these patients produce a highly viscous mucus in the lung [Ref. 9], making expectoration of pathogens less effective. Consequently, the patients can suffer from repeated infections above all with bacteria. After repeated treatment with antibiotics, Pseudomonas aeruginosa often remains. P. aeruginosa is a bacterium that is typically multiresistant against antibiotics [Ref. 10]. Inhalations with haloamines can be applied successfully to treat such life-threatening infections because haloamines are active against multiresistant pathogens and do not induce resistance. NCT is one exemplary haloamine that can be employed in compositions and methods according to the invention to treat bronchopulmonary infections in patients suffering from cystic fibrosis.

Another exemplary bronchopulmonary application for haloamines is the treatments for patients who are predisposed to bronchopulmonary infections. For example, treatment for immunocompromised patients (e.g., AIDS) or immunosuppressed patients (e.g., organ transplant recipients or cancer patients) can be performed with haloamines (e.g., NCT). In general, today, cancer is treated with immunosuppressive drugs. Particularly, hematological malignancies (e.g., leukemia) sometimes can be cured only by strong immunosuppressive drugs and irradiation. Because of the toxicity of this therapy to the bone marrow, bone marrow transplantation is needed immediately after this treatment. Because of the depletion of the white blood cells, these patients are extremely prone to life-threatening infections, particularly airway infections including the bronchopulmonary system, within the first few weeks after the transplantation during their hospitalization [Refs. 11-13]. Bacterial infections usually can be managed with antibiotics, but fungal infections which occur a few days after bacterial ones are mostly life-threatening due to their higher resistance against antifungal medication [Refs. 14,15]. Therefore, prophylaxis of fungal infections is discussed and performed in part with intravenous drug application. Inhalative prophylaxis with haloamines (e.g., NCT) can cover a comprehensive spectrum of pathogens and, moreover, avoid many of the side effects of intravenous drugs.

Yet another exemplary bronchopulmonary application for haloamines is the treatment and prevention of ventilator-associated infections in critically ill patients who need artificial ventilation. Infections of the bronchopulmonary system are difficult to avoid if continuous ventilation is needed for long time-periods. Prophylaxis with, for example, a daily inhalation with a haloamine can reduce the number of such infections significantly. For example, a treatment could include a dose of a haloamine (e.g., NCT) for a duration of time (e.g., several minutes daily).

After adaption of the dose, a combination of NCT and ammonium chloride can be used in all of the foregoing indications and treatments.

N-halogenated and N,N-dihalogenated compounds related to the current disclosure can be used in place of NCT for treating, preventing, and prophylaxis of the various bronchopulmonary infections discussed hereinabove.

### EXAMPLES

### Example 1

Anesthetized pigs inhaled test solutions of 1% NCT (n = 7), 5% NCT (n = 6), or 1% NCT plus 1% ammonium chloride (n = 6), and 0.9% saline solution as a control (n = 7), respectively. Applications were performed every hour over a four-hour period, i.e., 4 inhalations in total, with 5 mL each.

Arterial partial pressure of oxygen (PaO₂) values at baseline were within normal range in all animals without any intergroup difference (97 mmHg ± 7.6), reflecting healthy, anesthetized and ventilated animals.

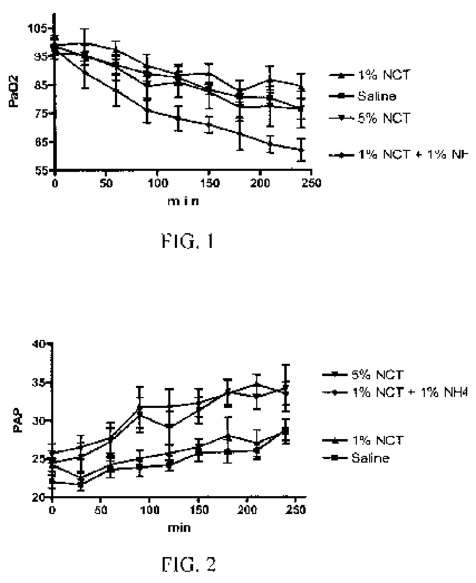

PaO₂ decreased significantly over the observation period of 4 hours in all animals (FIG. 1). No difference in PaO₂ to controls was seen in those animals receiving 1% NCT or 5% NCT, whereas inhalation with 1% + 1% NCT/ammonium chloride led to significantly lower PaO₂ values at the 4 hours measurement (62 mmHg ± 9.6 vs. 76 mmHg ± 9.2, p = 0.014). These results are presented in FIG. 1.

### Example 2

Anesthetized pigs inhaled test solutions of 1% NCT (n = 7), 5% NCT (n = 6), or 1% NCT plus 1% ammonium chloride (n = 6), and 0.9% saline solution as a control (n = 7), respectively. Applications were performed every hour within four hours, i.e., 4 inhalations in total, with 5 mL each.

Alveolo-arterial difference of oxygen partial pressure (AaDO₂) is a measure for intact oxygen transfer in the lung. The lower the values the better is the oxygen exchange. AaDO₂ increased in all animals during the experimental period and was highest in the 1% + 1 % NCT/ammonium chloride group (9.0 ± 7.2 to 45.7 ± 8.73, p = 0.00014). Compared to the controls, AaDO₂ was significantly higher in the 1%+ %NCT/ammonium chloride group (p = 0.00386), and not different in the 5% NCT group (p = 0.99). In sharp contrast and surprisingly, AaDO₂ was even lower than in controls in the 1% NCT group (21.0 ± 13.09 vs. 33.7 ± 9.03, p = 0.016) at the 4-hour measurement.

### Example 3

Anesthetized pigs inhaled test solutions of 1% NCT (n = 7), 5% NCT (n = 6), or 1% NCT plus 1% ammonium chloride (n = 6), and 0.9% saline solution as a control (n = 7), respectively. Applications were performed every hour within four hours, i.e., 4 inhalations in total, with 5 mL each. Heart rate at the 4 hours measurement was not significantly different from controls in the 1% NCT and the 5% NCT group, whereas it significantly increased in the 1% + 1% NCT/ammonium chloride group (104/min ± 5.4 to 136/min ± 16.9, P = 0.00002), resulting in a significant difference to controls at the 4 hours measurement (p = 0.00036). Systemic arterial pressure remained constant within physiological range over the entire experimental period in all animals alike without any inter-or intra-group differences.

### Example 4

Anesthetized pigs inhaled test solutions of 1% NCT (n = 7), 5% NCT (n = 6), or 1% NCT plus 1% ammonium chloride (n = 6), and 0.9% saline solution as a control (n = 7), respectively. Applications were performed every hour over a four-hour period of time, i.e., 4 inhalations in total, with 5 mL each.

Pulmonary artery pressure increased to some extend in all animals and reached significantly higher values in the 1% + 1% NCT/ammonium chloride and the 5% NCT group compared to controls. These results are presented in FIG. 2.

### Example 5

This example demonstrates the administration of a pharmaceutical acceptable formulation of a compound related to the current disclosure, having the formula: 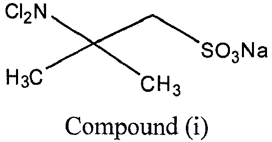 in accordance with the methods of the current disclosure. Anesthetized pigs inhale 1% (n = 7) or 5% (n = 6) aqueous solutions of Compound (i), or a 1% aqueous solution of Compound (i) plus 1% ammonium chloride (n = 6), and 0.9% saline solution is used as a control (n = 7). The solutions are administered by nasal administration every hour over a four-hour period, i.e., 4 inhalations in total, with 5 mL each. Arterial partial pressure of oxygen (PaO₂), Alveolo-arterial difference of oxygen partial pressure (AaDO₂), heart rate, systemic arterial pressure, and pulmonary artery pressure is measured during the 4 hour period.

### References

1. Dijkman JH, Vooren PH, Kramps JA. Occupational asthma due to inhalation of chloramine-To I. Clinical observations and inhalation-provocation studies. Int Arch Allergy Appl Immunol 1981; 64: 422-7.
2. Kramps JA, van Toorenenbergen AW, Vooren PH, Dijkman JR. Occupational asthma due to inhalation of chloramine-T. II. Demonstration of specific IgE antibodies. Int Arch Allergy Appl Immunol1981; 64: 428-38.
3. Gottardi W, Nagl M. Chemical properties of N-chlorotaurine sodium, a key compound in the human defence system. Arch Pharm Pharm Med Chem 2002; 335: 411-21.
4. Nagl M, Gottardi W. Enhancement of the bactericidal efficacy of N-chlorotaurine by inflammation samples and selected N-H compounds. Hyg Med 1996; 21: 597-605.
5. Nagl M, Larcher C, Gottardi W. Activity of N-chlorotaurine against herpes simplex- and adenoviruses. Antiviral Res 1998; 38: 25-30.
6. Nagl M, Gruber A, Fuchs A, Lell C, Lemberger EM, Borg-von Zepelin M, Wuerzner R Impact of N-chlorotaurine on viability and production of secreted aspartyl proteinases of Candida spp. Antimicrob Agents Chemother 2002; 46: 1996-9.
7. Yazdanbakhsh M, Eckmann CM, Roos D. Killing of schistosomula by taurine chloramine and taurine bromamine. Am J Trop Med Hyg 1987; 37: 106-10.
8. Nagl M & Gottardi W. Rapid killing of Mycobacterium terrae by N-chlorotaurine in presence of ammonium is caused by the reaction product monochloramine. J Pharm Pharmacol1998; 50: 1317-20.
9. Fuller MD, Thompson CH, Zhang ZR, Freeman CS, Schay E, Szakacs G, Bakos E, Sarkadi B, McMaster D, French RJ, Pohl J, Kubanek J, McCarty NA. State-dependent Inhibition of Cystic Fibrosis Transmembrane Conductance Regulator Chloride Channels by a Novel Peptide Toxin. J BioI Chem 2007; 282: 37545-55.
10. Cohn LA, Weber A, Phillips T, Lory S, Kaplan M, Smith A. Pseudomonas aeruginosa infection of respiratory epithelium in a cystic fibrosis xenograft model. J Infect Dis 2001; 183: 919-27.
11. Krowka MJ, Rosenow EC, III, Hoagland He. Pulmonary complications of bone marrow transplantation. Chest 1985; 87: 237-46.
12. Lass-Florl C, Gunsilius E, Gastl G, Englisch M, Koch G, Ulmer H, Dierich MP, Petzer A. Fungal colonization in neutropenic patients: a randomized study comparing itraconazole solution and amphotericin B solution. Ann Hematol2003; 82: 565-9.
13. Lass-Florl C, Aigner J, Gunsilius E, Petzer A, Nachbaur D, Gastl G, Einsele H, Loffler J, Dierich MP, Wurzner R Screening for Aspergillus spp. using polymerase chain reaction of whole blood samples from patients with haematological malignancies. Br J Haematol 2001; 113: 180-4.
14. Eschertzhuber S, Velik-Salchner C, Hoermann C, Hoefer D, Lass-Florl C. Caspofunginresistant Aspergillus flavus after heart transplantation and mechanical circulatory support: a case report. Transpl Infect Dis 2007*;*
15. Lass-Florl C, GriffK, Mayr A, Petzer A, Gastl G, Bonatti H, Freund M, Kropshofer G, Dierich MP, Nachbaur D. Epidemiology and outcome of infections due to Aspergillus terreus: 10-year single centre experience. Br J Haematol2005; 131: 201-7.
16. van Gelder, N.M. and Bowers, RJ., "Synthesis and characterization of N,N-dichlorinated amino acids: taurine, homotaurine, GABA and L-leucine," Neurochemical Research, 2001, 26(6): 575-8
17. Gottardi W. et al., "N,N-Dichlorotaurine: chemical and bactericidal properties," Arch Pharm Med Chem 2005; 338: 473-83.
18. Gottardi W. et al., "N-Chlorotaurine and ammonium chloride: An antiseptic preparation with strong bactericidal activity," Int. J. Pharmaceut. 2007, 335(1-2): 32-40.
19. Grisham et al.: 1984 Chlorination of endogenous amines by isolated neutrophils. Journal of Biological Chemistry 259 10404-10413
20. Weiss: 1989 Tissue destruction by neutrophils. New England Journal of Medicine 320 365-376.
21. Kontny et al.: 1999 Taurine chloramine inhibition of cell proliferation and cytokine production by rheumatoid arthritis fibroblast-like synoviocytes. Arthritis and Rheumatism 42 2552-2560.
22. Park et al.: 2000 Regulation of nitric oxide induced by mycobacteriallipoarabinomannan in murine macrophages: effects of interferon-beta and taurine-chloramine. International Journal of Leprosy and Other Mycobacterial Diseases 68444-451.
23. Marcinkiewicz: 2003 Prostanoids and MPO-halide system products as a link between innate and adaptive immunity. Immunology Letters 89 187-191.
24. Nagl et al.: 2001 Enhanced fungicidal activity of N-chlorotaurine in nasal secretion. Journal of Antimicrobial Chemotherapy 47 871-874.
25. Nagl et al.: 2000 Bactericidal activity of micromolar N-chlorotaurine -evidence for its antimicrobial function in the human defence system. Antimicrobial Agents and Chemotherapy 44 2507-2513.
26. Nagl et al.: 1998 Tolerance of N-chlorotaurine, an endogenous antimicrobial agent, in the rabbit and human eye -a phase I clinical study. Journal of Ocular Pharmacology and Therapeutics 14 283-290.
27. Romanowski et al.: 2006 N-Chlorotaurine is an Effective Antiviral Agent against Adenovirus In Vitro and in the Ad5/NZW Rabbit Ocular Model. Investigative Ophthalmology & VisualScience 47 2021-2026.
28. Neher et al.: 2004 Acute Otitis Externa: Efficacy and Tolerability of N-Chlorotaurine, a Novel Endogenous Antiseptic Agent. Laryngoscope 114850-854.
29. Neher et al.: 2005 Tolerability of N-Chlorotaurine in Chronic Rhinosinusitis Applied via Yamik Catheter. Auris Nasus Larynx 32 359-364.
30. Nagl et al.: 2003 Tolerability and efficacy of N-chlorotaurine compared to chloramine T for treatment of chronic leg ulcers with purulent coating. Br J Dermatol 149 590-597.
31. Unterberger et al.: 2001: N-chlorotaurine -local antibacterial therapy in urinary tract infections by omniresistant Pseudomonas aeruginosa. 21. Annual Conference of the Arbeitsgemeinschaft Neurologische Intensivmedizin (ANIM), Innsbruck.
32. Neher et al.: 2001 N-chlorotaurine, a novel endogenous antimicrobial agent: tolerability tested in a mouse model. Arch. Otolaryngol. Head Neck Surg. 127 530-533.
33. Neher et al.: 2004 Tolerability of N-chlorotaurine in the guinea pig middle ear -a pilot study using an improved application system. Annals of Otology, Rhinology & Laryngology 113 76-81.
34. Verdrengh et al.: 2005 Inhibition of septic arthritis by local administration of taurine chloramine, a product of activated neutrophils. Journal of Rheumatology 32 15131517.
35. Dychdala 2001: Chlorine and chlorine compounds. In: Disinfection, Sterilization and Preservation, 135-158 Lippincott Williams & Wilkins, Philadelphia.

## Claims

1. A composition comprising N-chlorotaurine or N,N-dichlorotaurine for use in treating or preventing a bronchopulmonary infection in a mammal, wherein N-chlorotaurine or N,N-dichlorotaurine is to be administered by inhalation.

2. The composition for the use according to claim 1, **characterized in that** the bronchopulmonary infection treated or prevented is selected from the group consisting of obstructive pulmonary disease, cystic fibrosis, and ventilator-associated infections.

3. A composition according to claim 1, **characterized in that** said composition comprises 0.1% weight per volume to 5% weight per volume N-chlorotaurine for use in treating a bronchopulmonary infection in a mammal, wherein N-chlorotaurine is to be administered by inhalation.

4. A composition according to claim 1, **characterized in that** said composition comprises 1% weight per volume N-chlorotaurine for use in treating a bronchopulmonary infection in a mammal, wherein N-chlorotaurine is to be administered by inhalation.

## Patentansprüche

1. Eine Zusammensetzung, umfassend N-Chlortaurin oder N,N-Dichlortaurin zur Verwendung bei der Behandlung oder Vorbeugung einer bronchopulmonalen Infektion bei einem Säugetier, wobei N-Chlortaurin oder N,N-Dichlortaurin durch Inhalation zu verabreichen ist.

2. Die Zusammensetzung zur Verwendung entsprechend Anspruch 1, dadurch charakterisiert, dass die bronchopulmonale Infektion, die behandelt oder vor der vorgebeugt wird, aus der Gruppe bestehend aus obstruktiven Lungenerkrankung, zystischer Fibrose und Ventilator-assoziierten Infektionen ausgewählt wird.

3. Eine Zusammensetzung entsprechend Anspruch 1, dadurch charakterisiert, dass jene Zusammensetzung 0,1% Gewicht pro Volumen bis 5% Gewicht pro Volumen N-Chlortaurin zur Verwendung bei der Behandlung einer bronchopulmonalen Infektion bei einem Säugetier umfasst, wobei N-Chlortaurin durch Inhalation zu verabreichen ist.

4. Eine Zusammensetzung entsprechend Anspruch 1, dadurch charakterisiert, dass jene Zusammensetzung 1% Gewicht pro Volumen N-Chlortaurin zur Verwendung bei der Behandlung einer bronchopulmonalen Infektion bei einem Säugetier umfasst, wobei N-Chlortaurin durch Inhalation zu verabreichen ist.

## Revendications

1. Composition comprenant de la N-chlorotaurine ou de la N,N-dichlorotaurine pour une utilisation dans le traitement ou la prévention d'une infection broncho-pulmonaire chez un mammifère, dans laquelle la N-chlorotaurine ou la N,N-dichlorotaurine doit être administrée par inhalation.

2. Composition pour l'utilisation selon la revendication 1, **caractérisée en ce que** l'infection broncho-pulmonaire traitée ou prévenue est choisie dans le groupe constitué de la maladie pulmonaire obstructive, de la mucoviscidose, et d'infections associées au ventilateur.

3. Composition selon la revendication 1, **caractérisée en ce que** ladite composition comprend 0,1 % en poids par volume à 5 % en poids par volume de N-chlorotaurine pour une utilisation dans le traitement d'une infection broncho-pulmonaire chez un mammifère, dans laquelle la N-chlorotaurine droit être administrée par inhalation.

4. Composition salon la revendication 1, **caractérisée en ce que** ladite composition comprend 1 % en poids par volume de N-chlorotaurine pour une utilisation dans le traitement d'une infection broncho-pulmonaire chez un mammifère, dans laquelle la N-chlorotaurine doit être administrée par inhalation.
